# EUROPEAN PATENT SPECIFICATION

(11) **EP 1 167 340 B1**
(45) Date of publication and mention of the grant of the patent: **17.12.2003**
(21) Application number: 01201646.5
(22) Date of filing: 07.05.2001
(51) Int. Cl.: C07C 69/52, C12P 7/64, C07C 67/03, A23D 7/00, C11C 3/00

(54) **Cla-esters**
Cla-ester
Cla-esters

(30) Priority: 19.06.2000 EP 00305185
(43) Date of publication of application: 02.01.2002
(73) Proprietor: LODERS CROKLAAN B.V., 1521 AZ Wormerveer (NL)
(72) Inventor: Barclay, Scott, Unilever Research Colworth, Sharnbrook, Bedfordshire MK44 1LQ (GB); Rakowski, Krysztof Piotr, Loders Croklaan B.V., 1521 AZ Wormerveer (NL); Taran, Victoria, Loders Croklaan B.V., 1521 AZ Wormerveer (NL)
(74) Representative: Stevens, Ian Edward

(56) References cited:
- EP-A- 1 022 306
- WO-A-99/32105
- GARCIA H S ET AL: "ENRICHEMENT OF BUTTEROIL WITH CONJUGATED LINOLEIC ACID VIA ENZYMATIC INTERESTERIFICATION (ACIDOLYSIS) REACTIONS" BIOTECHNOLOGY LETTERS, KEW, SURREY, GB, vol. 20, no. 4, April 1998 (1998-04), pages 393-395, XP001027395 ISSN: 0141-5492

## Description

Esters from CLA (=reaction product of conjugated linoleic acid and an alcohol) are known from eg WO 97/18320 or WO 99/32105. However the esters disclosed in WO 97/18320 are mainly the octylesters and these esters have the disadvantage that the alcohol residue is not food allowable and that therefore the use of these esters in foods is a problem. In this same document also glycerol esters of CLA isomers are disclosed which can be made from glycerol and free CLA by esterification. However during this esterification the enrichment in a specific CLA isomer (in general c9t11 or t10c12 CLA) is low or the reaction rate and or yield for this conversion is very low. Moreover the products so obtained often are not very active for their desired health effect. Another problem is that the esters disclosed in WO '105 often are derived from an alcohol that, although being food allowable is not readily available or is difficult to remove from the reaction mixture (eg tocopherol alcohol or ascorbyl alcohols or retinyl alcohols).

We studied whether we could find a solution for above problems. This study resulted in the finding of new esters of CLA isomers that can be made easily in good yields in relatively short times while the products obtained often displayed high enrichment rates in specific CLA-isomers (in particular in c9t11 and/or t10c12 isomers). Moreover the alcohols used herefore are food allowable and easily available and can be separated easily from the crude reaction mix resulting from the partial conversion of the free CLA with the alcohol.

Moreover we found that these esters displayed good health properties and excellent taste properties. In particular the taste of the esters was improved compared to the taste of the free acids.
Therefore our invention concerns in the first instance with esters of conjugated linoleic acid (= CLA) and an alcohol wherein the alcohol is selected from food allowable terpene alcohols or food allowable sesquiterpene alcohols.
In particular the use of the alcohols with general formula I (cf formula sheet ), wherein :
R1 = H, or forms together with R4 a C-C bond,
R2 = H or CH3
R3 = H, CH3 or OH
R4 = H or forms together with R1, or with R6 or with R14 a C-C bond, or forms together with R9 a -C- bridge or forms together with R9 a 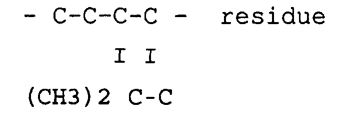
R5 = H or forms together with R13 or with R14 a C-C bond
R6 = H or forms together with R4 a C-C bond
R7 = H, or OH
R8 = H or OH or forms together with R10 a C-C bond
R9 = H,or forms together with R4 a -C- bridge or a 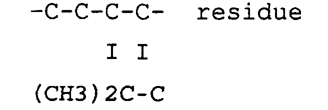
R10 = H or forms together with R8 or with R12 a C-C bond
R11 = H, i-propyl, i-propenyl, CH3 or forms together with R4 a -C(CH3)2- bridge
R12 = H or forms together with R10 a C-C bond
R13 = H, OH, or -C(CH3)=C-C(OH)- or forms together with R5 a C-C bond
R14 = H, or forms together with R4 or R5 a C-C bond lead to excellent results

In particular the invention concerns with esters derived from a food allowable alcohol selected from the group consisting of menthol, isopulegol, methenol, carveol, carvomenthenol, carvomenthol, isobornylalcohol, caryophyllenealcohol, geraniol, farnesol and citronellol. These alcohols are all food allowed, easily available and give high enrichment in specific CLA isomers when converted partially with free CLA (in particular with a 50:50 mix of c9t11 and t10c12 CLA) along an enzymic route. Moreover the esters display good activity with respect to anti inflammatory and anti diabetic or insuline resistance effects and taste far better than the free CLA where they are derived from.
The esters can be obtained easily in a form wherein the esters comprise more than 80 wt %, preferably more than 90 wt % and in particular more than 95 wt % of c9t11 plus t10c12 CLA isomers. Depending on the conversion that is applied the esters can be obtained as a mix wherein the c9t11 and t10c12 CLA-isomers are present in a weight ratio of 50:50, or as a mix wherein the c9t11 : t10c12 isomers are present in a weight ratio of more than 70 : 30, in particular more than 80 : 20. The esters of the enriched isomer mix have the advantage that the mix mainly will display the specific health effects from the specific isomer present in high contents while the other specific health effects of the other isomer hardly will be noticed. This enables us to dose more specifically to achieve a specific health effect, while also the dosage of the CLA compound can be less which is of particular advantage if the esters are applied as food supplement because this will limit the size of the capsules and the smaller the capsules can be, the easier they can be swallowed by the consumer.

A particular preference exists for a composition comprising esters of CLA and menthol wherein more than 80 wt %,preferably more than 90 wt % of the CLA residue of the esters consists of c9t11-CLA, while less than 2 wt % of the total CLA-residue consists of CLA-isomers other than c9t11 and t10c12 and the esters contain less than 5 wt % preferably less than 3 wt% of free fatty acids.
Administering of these products means that potentially negative side effects of other components can be avoided.

Our invention further concerns a process for the preparation of esters of conjugated linoleic acid (=CLA) and a food allowable alcohol as defined above, wherein free conjugated linoleic acid is reacted with the food allowable alcohol in the presence of less than 1.8 wt % water and a lipase. This process will result in esters that can be used in food products as both components of the products are food allowable.
In the instance that an ester is desired wherein higher contents of a specific CLA-isomer is present the above process can be adapted to a process wherein a food allowable alcohol with the formula as defined above is reacted with a mix of free CLA with about 50 % of both main isomers (c9t11 and t10c12) and wherein the reaction is performed by partially converting this mixture of c9t11 and t10c12 CLA in the presence of an enzyme than can discriminate between c9t11 and t10c12-CLA isomers and which enzyme preferably is cand rugosa lipase,whereupon CLA-esters enriched in c9t11 CLA-isomer are isolated and free fatty esters enriched in t10c12 CLA isomer are isolated as non-converted reactant.
Other enzymes that can be applied are lipase D; lipase QL; lipase-SL; mucor miehei lipase, optionally on a support such as duolite; cand antartica lipase and lipozyme.

The different products can be obtained by working up the crude reaction product as follows:
- first add a diluted base to the crude reaction mixture
- then separate the water phase and the organic phase
- followed by washing of the organic phase and adjusting the pH of this phase to pH 5-7
- and finally by removal of the water from this phase, preferably by distillation.
   By this route esters are obtained enriched in the c9t11 CLA isomer.

To obtain a free CLA product enriched in the t10c12 isomer the procedure is as follows:
- the water phase of the reaction product obtained according to above process is acidified to a pH < 3.0, preferably < 1.5 ,
- whereupon the mixture obtained is separated in an upper layer and a residue and
- the upper layer is washed with water and the water is removed therefrom.

According to another aspect of our invention our invention also concerns food products comprising a fat and a good tasting health component wherein the taste and health component is an ester or ester composition according to the invention. The food products are specifically selected from the group consisting of spreads (10-90% fat contents); dressings, mayonnaise, cheese, ice cream, ice cream coatings; confectionery coatings or fillings; sauces and culinary products. These food products suitably contain 0.5 to 20 wt % of our novel CLA esters or ester composition.

Our novel esters can also be used for the preparation of triglycerides containing CLA residues. Herefore an ester or ester composition according to the invention can be converted with glycerol or with a vegetable oil in the presence of a base or an enzyme while in the instance that glycerol is converted the liberated alcohol residue from the esters of the CLA-esters is removed from the reaction mix during the conversion. This removal of liberated alcohol can be done by vacuum destillation or by molecular distillation.

According to a last embodiment our invention also concerns the use of our CLA esters as a good tasting health agent, in particular having anti-inflammatory or anti-diabetic or insulin resistant properties.

### Examples

### 1) CLA menthol esters:

| | |
|---|---|
| 2.5 g | CLA 50:50, 80% main isomers (cis9,t11, t10,c12) |
| 0.02 g | demin water |
| 0.4 g | menthol |
| 0.026 g | *Candida rugosa* lipase |
| T 38 °C | |

### Reaction time 22 hours

Mix well 2.5 g CLA with 0.02 g water. Add 0.4 g menthol and mix very well for 30 min in shaker at 38 °C and 200 rpm. Add this mixture to 0.026 g *Candida rugosa* lipase; mix everything very well. Put the mixture in the shaker at 38 °C and 200 rpm. After 22 hours add to the reaction mixture 30 ml isooctane/ethanol (1:1) and 33 ml 0.2 N NaOH. Mix very well. Centrifuge for 5 min at 3500 rpm. Separate the organic phase from the aqua phase. Wash the organic phase 3x 100 ml of demin water and evaporate the solvent.

Add aqua phase to 50 ml H₂SO₄ (1:10) and 30 ml isooctane. Remove the organic phase and wash 3x 100 ml of demin water. Evaporate the solvent. The analysis of FFA and menthol esters fractions are shown in table below:

| | Fame of CLA FFA | Fame of CLA menthol esters |
|---|---|---|
| C16:0 | 2.4 | 2.3 |
| C16:1 | 0.1 | 0.1 |
| C18:0 | 2.5 | 1.7 |
| CLA,t,t | 2.8 | 0.6 |
| CLAc9,c11 | 1.1 | 0.4 |
| CLAc10,c12 | 1.3 | 0.4 |
| CLAc11,c13 | 0.6 | 0.9 |
| C18:1,t | 1.1 | 0.0 |
| C18:1,c | 10.8 | 13.7 |
| CLA-OX | 0.0 | 0.2 |
| C18:2,t | 0.4 | 0.6 |
| C18:2c | 2.1 | 3.6 |
| C20:0 | 0.7 | 0.1 |
| C20:1 | 0.3 | 0.0 |
| C22:0 | 0.1 | 0.1 |
| CLA,c9,t11 | 28.1 | **62.1** |
| CLA,t10,c12 | **45.5** | 13.2 |
| Ratio c9,t11: t10,c12 | **38:62** | **83:17** |
| Conversion | 32 % | |
| FFA, % | 99.3 | 1.85 |

### 2) CLA geraniol ester:

| | |
|---|---|
| 20 g | CLA 50:50 |
| 3.64 g | geraniol |
| 0.24 g | demin water |
| 0.2 g | *Candida rugosa* lipase |
| T 38 °C | |

### Reaction time 1 hour

The CLA, geraniol and water were mixed in a vial and incubated in a shaking water bath at 38 °C. After 30 minutes the lipase was added, mixed by shaking and the vial replaced in the water bath. After 1 hour the reaction mixture was diluted with an equal volume of cold hexane and filtered through a 0.45 µm PTFE membrane to remove the enzyme. After evaporation of the solvent 17 g of the remaining oil was dissolved in 100 ml of hexane and mixed with 100 ml of demin water in which 2.84 g of sodium hydroxide had been dissolved. After vigorous shaking, 10 ml of ethyl alcohol was added to ease the separation of the layers.
The aqueous phase was removed and added to 100 ml of a 20% hydrochloric acid solution and 100 ml of hexane and shaken vigorously. The organic layer was removed and washed with 3x 100 ml of demin water, 100 ml of brine and the solvent evaporated to leave 14 g of oil comprising 85 % FFA.
The organic phase was washed with 2x 100 ml of hot alkaline demin water, 100 ml of demin water and 100 ml of brine. The dry organic phase was passed through a column of basic alumina to remove any traces of remaining FFA and the solvent evaporated to yield 3 g of oil.

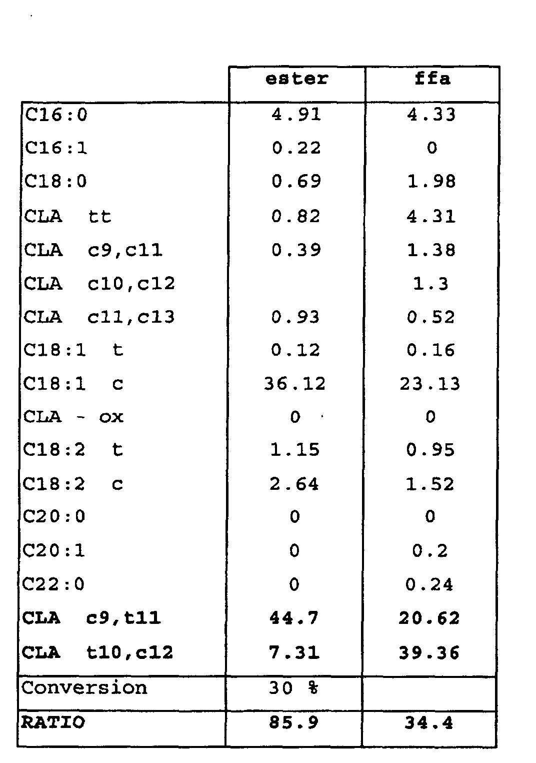

The two product fractions were examined by TLC and the respective bands for FFA and ester removed and worked up FAME analysis.

### 3) CLA citronellol ester:

| | |
|---|---|
| 5 g | CLA |
| 1.1 g | citronellol |
| 0.06 g | demin water |
| 0.1 g | *Candida rugosa* lipase |
| T 30 °C | |

### Reaction time 30 minutes

The CLA, citronellol and water were mixed in a vial and incubated in a shaking water bath at 30 °C. After 30 minutes the lipase was added, mixed by shaking and the vial replaced in the water bath. After 30 minutes the reaction mixture was filtered through a 0.45 µm PTFE membrane to remove the enzyme. The reaction had reached 20 % conversion.

The reaction mixture was analysed by TLC. The ester bands was worked up for FAME analysis.

| | |
|---|---|
| C16:0 | 2.89 |
| C16:1 | 0.21 |
| C18:0 | 0.71 |
| C18:1 | 9.98 |
| C18:2 | 1.47 |
| C20:0 | 0.47 |
| C20:1 | 0.28 |
| C22:1 | 0.41 |
| CLA c9,t11 | 39.65 |
| CLA t10,c12 | 7.65 |
| CLA c,c | 1.78 |
| CLA t,t | 6.99 |
| Conversion | 20 % |
| **Ratio** | **84:16** |

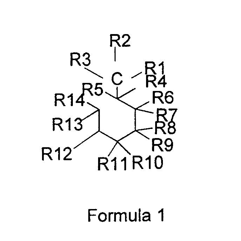

## Claims

1. Esters of conjugated linoleic acid (=CLA) and an alcohol, wherein the alcohol is a food allowable terpene alcohol or a food allowable sesquiterpene alcohol.

2. Esters of conjugated linoleic acid and an alcohol according to claim 1 wherein the food allowable terpene alcohol or food allowable sequiterpene alcohol is selected from the alcohols with general formula I , wherein : 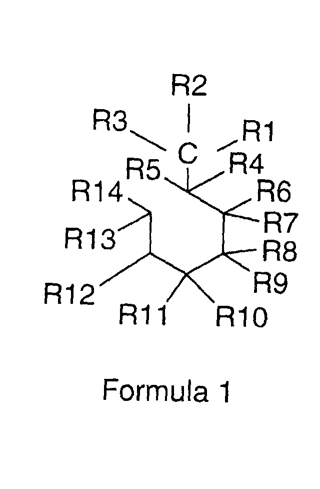
R1 = H, or forms together with R4 a C-C bond,
R2 = H or CH3
R3 = H, CH3 or OH
R4 = H or forms together with R1, or with R6 or with R14 a C-C bond, or forms together with R9 a -C- bridge or forms together with R9 a 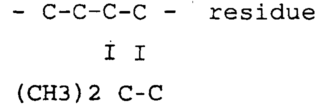
R5 = H or forms together with R13 or with R14 a C-C bond
R6 = H or forms together with R4 a C-C bond
R7 = H, or OH
R8 = H or OH or forms together with R10 a C-C bond
R9 = H,or forms together with R4 a -C- bridge or a 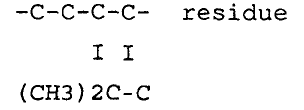
R10 = H or forms together with R8 or with R12 a C-C bond
R11 = H, i-propyl, i-propenyl, CH3 or forms together with R4 a -C(CH3)2- bridge
R12 = H or forms together with R10 a C-C bond
R13 = H, OH, or-C(CH3)=C-C(OH)- or forms together with R5 a C-C bond
R14 = H, or forms together with R4 or R5 a C-C bond

3. Esters according to claims 1-2, wherein the food allowable alcohol is selected from the group consisting of menthol, isopulegol, methenol, carveol, carvomenthenol, carvomenthol, isobornylalcohol, caryophyllenealcohol, geraniol, farnesol and citronellol.

4. Esters according to claims 1-3 wherein the CLA residue of the esters comprise more than 80 wt % of c9t11 plus t10c12 CLA isomers.

5. Esters according to claim 4 wherein the c9t11 and t10c12 CLA-isomers are present in a weight ratio of 50:50.

6. Esters according to claim 4 wherein the c9t11 : t10c12 isomer weight ratio in the CLA-esters is more than 70 : 30.

7. Composition consisting of esters of CLA and menthol wherein more than 80 wt % of the CLA residue of the esters consists of c9t11-CLA, while less than 2 wt % of the total CLA-residue consists of CLA-isomers other than c9t11 and t10c12 and the esters contain less than 5 wt % of free fatty acids .

8. Process for the preparation of esters of conjugated linoleic acid (=CLA) and an alcohol as defined in claim 1, wherein free conjugated linoleic acid is reacted with a food allowable alcohol as defined in claim 1 in the presence of less than 1.8 wt % water and a lipase.

9. Process for the preparation of esters of CLA and an alcohol , wherein the esters obtained are enriched in c9t11-CLA and wherein the reaction is performed by partially converting a mixture of c9t11 and t10c12 CLA in a weight ratio of about 50:50 and a food allowable alcohol from the group as defined in claim 1 in the presence of an enzyme than can discriminate between c9t11 and t10c12-CLA isomers whereupon CLA-esters enriched in c9t11 CLA-isomer are isolated and free fatty acids enriched in t10c12 CLA isomer are isolated as non-converted reactant.

10. Process according to claims 9 and 10 wherein the crude reaction mixture obtained is worked up by :
- first adding a diluted base to the crude reaction mixture
- separating the water phase and the organic phase
- washing of the organic phase and adjusting the pH of this phase to pH 5-7
- removal of the water from this phase by distillation

11. Process for the preparation of a free CLA mixture enriched in t10c12-CLA wherein:
- the water phase of the reaction product obtained according to the process of claim 10 is acidified to a pH < 3.0, preferably < 1.5 ,
- whereupon the mixture obtained is separated in an upper layer and a residue and
- the upper layer is washed with water and the water is removed therefrom.

12. Food products comprising a fat and a health component wherein the health component is one or more of the esters according to claims 1 to 6 or the ester composition according to claim 7.

13. Food products according to claim 12 wherein the food product is selected from the group consisting of spreads (10-90% fat contents); dressings, mayonnaise, cheese, ice cream, ice cream coatings; confectionery coatings or fillings; sauces and culinary products.

14. Food products according to claims 12 and 13 wherein the food product contains on total product 0.5 to 20 wt % of the esters of CLA according to claims 1 - 6 or the ester composition according to claim 7.

15. Process for the preparation of triglycerides wherein at least one of the fatty acid residues is a conjugated linoleic acid residue wherein an ester according to claims 1 -6 or an ester composition according to claim 7 is converted with glycerol or with a vegetable oil in the presence of a base or an enzyme while in the instance that glycerol is converted the liberated alcohol residue from the esters of the CLA-esters is removed from the reaction mix during the conversion.

## Patentansprüche

1. Ester aus konjugierter Linolsäure (= CLA) und einem Alkohol, wobei der Alkohol ein nahrungsmittelgeeigneter Terpenalkohol oder ein nahrungsmittelgeeigneter Sesquiterpenalkohol ist.

2. Ester aus konjugierter Linolsäure und einem Alkohol gemäß Anspruch 1, wobei der nahrungsmittelgeeignete Terpenalkohol oder der nahrungsmittelgeeignete Sequiterpenalkohol ausgewählt ist aus den Alkoholen mit der allgemeinen Formel I, wobei: 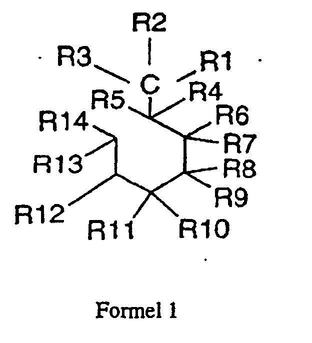
R1 = H, oder bildet zusammen mit R4 eine C-C-Bindung,
R2 = H oder CH3
R3 = H, CH3 oder OH
R4 = H oder bildet zusammen mit R1, oder mit R6 oder mit R14 eine C-C-Bindung, oder bildet zusammen mit R9 eine -C- Brücke oder bildet zusammen mit R9 einen 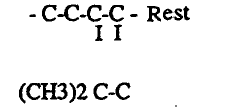
R5 = H oder bildet zusammen mit R13 oder mit R14 eine C-C Bindung
R6 = H, oder bildet zusammen mit R4 eine C-C-Bindung
R7 = H, oder OH
R8 = H oder OH oder bildet zusammen mit R10 eine C-C-Bindung
R9 = H, oder bilder zusammen mit R4 eine -C- Brücke oder einen 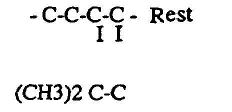
R10 = H oder bildet zusammen mit R8 oder mit R12 eine C-C Bindung
R11 = H, i-Propyl, i-Propenyl, CH3 oder bildet zusammen mit R4 ein -C(CH3)2- Brücke
R12 = H oder bildet zusammen mit R10 eine C-C-Bindung
R 13 = H, OH, oder -C(CH3)=C-C(OH)- oder bildet zusammen mit R5 eine C-C-Bindung
R14 = H, oder bildet zusammen mit R4 oder R5 eine C-C-Bindung.

3. Ester gemäß den Ansprüchen 1-2, wobei der nahrungsmittelgeeignete Alkohol ausgewählt ist aus der Gruppe, bestehend aus Menthol, Isopulegol, Methenol, Carveol, Carvomenthenol, Carvomenthol, Isobornylalkohol, Caryophyllenealkohol, Geraniol, Farnesol und Citronellol.

4. Ester gemäß den Ansprüchen 1-3, wobei der CLA-Rest der Ester mehr als 80 Gew.-% c9t11-plus t10c12-CLA-Isomere umfasst.

5. Ester gemäß Anspruch 4, wobei die c9t11- und t10c12-CLA-Isomere in einem Gewichtsverhältnis von 50 : 50 vorliegen.

6. Ester gemäß Anspruch 4, wobei das c9t11 : t10c12-Isomergewichtsverhältnis in den CLA-Estern größer ist als 70:30.

7. Zusammensetzung, bestehend aus Estern aus CLA und Menthol, wobei mehr als 80 Gew.-% des CLA-Rests der Ester aus c9t11-CLA besteht, während weniger als 2 Gew.-% des gesamten CLA-Rests aus anderen CLA-Isomeren als c9t11 und t10c12 bestehen und die Ester weniger als 5 Gew.-% freie Fettsäuren enthalten.

8. Verfahren zur Herstellung von Estern aus konjugierter Linolsäure (=CLA) und einem Alkohol wie in Anspruch 1 definiert, wobei freie konjugierte Linolsäure mit einem nahrungsmittelgeeigneten Alkohol wie in Anspruch 1 definiert in Anwesenheit von weniger als 1,8 Gew.-% Wasser und einer Lipase zur Reaktion gebracht wird.

9. Verfahren für die Herstellung von Estern aus CLA und einem Alkohol, wobei die erhaltenen Ester in c9t11-CLA angereichert sind und die Reaktion ausgeführt wird, indem ein Gemisch aus c9t11- und t10c12-CLA in einem Gewichtsverhältnis von etwa 50:50 und einem nahrungsmittelgeeigneten Alkohol aus der Gruppe wie in Anspruch 1 definiert in Anwesenheit eines Enzyms, das zwischen c9t11- und t10c12-CLA-Isomeren unterscheiden kann, teilweise umgewandelt wird, woraufhin in c9t11-CLA-Isomer angereicherte CLA-Ester isoliert werden und in t10c12-CLA-Isomer angereicherte freie Fettsäuren als nicht umgewandelter Reaktionspartner isoliert werden.

10. Verfahren gemäß den Ansprüchen 9 und 10, wobei das erhaltene rohe Reaktionsgemisch aufgearbeitet wird durch:
- Als Erstes Hinzufügen einer verdünnten Base zum rohen Reaktionsgemisch
- Separieren der Wasserphase und der organischen Phase
- Waschen der organischen Phase und Einstellen des pH dieser Phase auf pH 5-7
- Entfernen des Wassers aus dieser Phase durch Destillation

11. Verfahren für die Herstellung eines in t10c12-CLA angereicherten freien CLA-Gemischs, wobei:
- die Wasserphase des gemäß dem Verfahren von Anspruch 10 erhaltenen Reaktionsprodukts auf einen pH < 3,0 angesäuert wird, vorzugsweise < 1,5 ,
- woraufhin das erhaltene Gemisch in eine obere Schicht und einen Rest separiert wird und
- die obere Schicht mit Wasser gewaschen und das Wasser davon entfernt wird.

12. Nahrungsmittelprodukte, umfassend ein Fett und eine Gesundheitskomponente, wobei es sich bei der Gesundheitskomponente um einen oder mehrere der Ester gemäß den Ansprüchen 1 bis 6 oder die Esterzusammensetzung gemäß Anspruch 7 handelt.

13. Nahrungsmittelprodukte gemäß Anspruch 12, wobei das Nahrungsmittelprodukt ausgewählt ist aus der Gruppe, bestehend aus Aufstrichen (10-90 % Fettanteil); Dressings, Mayonnaise, Käse, Eiskrem, Eiskremüberzügen; Süßwarenüberzügen oder -füllungen; Soßen und Kochprodukten.

14. Nahrungsmittelprodukte gemäß den Ansprüchen 12 und 13, wobei das Nahrungsmittelprodukt bezogen auf das Gesamtprodukt 0,5 bis 20 Gew.-% der Ester aus CLA gemäß den Ansprüchen 1 - 6 oder der Esterzusammensetzung gemäß Anspruch 7 enthält.

15. Verfahren für die Herstellung von Triglyceriden, wobei mindestens eine der Fettsäurereste ein konjugierter Linolsäurerest ist, wobei ein Ester gemäß den Ansprüchen 1-6 oder eine Esterzusammensetzung gemäß Anspruch 7 mit Glycerin oder mit einem Pflanzenöl in Anwesenheit einer Base oder eines Enzyms umgewandelt wird, während im Fall der Umwandlung von Glycerin der freigesetzte Alkoholrest von den Estern der CLA-Ester aus dem Reaktionsgemisch bei der Umwandlung entfernt wird.

## Revendications

1. Esters d'acide linoléique conjugué (ALC) et d'un alcool, dans lesquels l'alcool est un alcool de terpène autorisé dans les produits alimentaires ou un alcool de sesquiterpéne autorisé dans les produits alimentaires.

2. Esters d'acide linoléique conjugué et d'un alcool selon la revendication 1, dans lesquels l'alcool de terpène autorisé dans les produits alimentaires ou l'alcool de sesquiterpène autorisé dans les produits alimentaires sont choisis parmi les alcools de formule générale I : 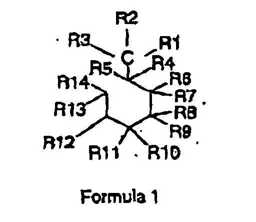 dans laquelle :
R1 = H ou forme conjointement avec R4 une liaison C-C,
R2 = H ou CH3
R3 = H, CH3 ou OH
R4 = H ou forme conjointement avec R1, ou avec R6 ou avec R14 une liaison C-C, ou forme conjointement avec R9 un pont, ou forme conjointement avec R9 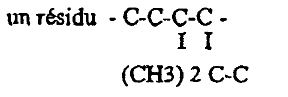
R5 = H ou forme conjointement avec R13 ou R14 une liaison C-C
R6 = H ou forme conjointement avec R4 une liaison C-C
R7 = H ou OH
R8 = H ou OH ou forme conjointement avec R10 une liaison C-C
R9 = H ou forme conjointement avec R4 un pont -C- ou 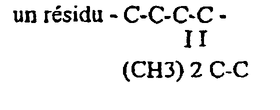
R10 = H ou forme conjointement avec R8 ou R12 une liaison C-C
R11 = H, i-propyle, i-propényle, CH3 ou forme conjointement avec R4 un pont -C(CH3) 2 -
R12 = H ou forme conjointement avec R10 une liaison C-C
R13 = H, OH ou C-(CH3) = C-C (OH) ou forme conjointement avec R5 une liaison C-C
R14 = H ou forme conjointement avec R4 ou R5 une liaison C-C.

3. Esters selon les revendications 1-2, dans lesquels l'alcool autorisé dans les produits alimentaircs est choisi parmi le groupe constitué de menthol, isopulégol, méthanol, carvéol, carvomenthénol, carvomenthol, alcool d'isobomyle, alcool de caryophyllène, géraniol, famesol et citronellol.

4. Esters selon les revendications 1-3, dans lesquels le résidu ALC des esters comprend plus de 80% en poids d'isomères ALC c9t11 et t10c12.

5. Esters selon la revendication 4, dans lesquels les isomères ALC c9t11 et t10c12 sont présents dans un ratio de poids de 50:50.

6. Esters selon la revendication 4, dans lesquels le ratio de poids isomère c9t11 : t10c12 dans les esters ALC est supérieur à 70:30.

7. Composition constituée d'esters d'ALC et de menthol, dans laquclle plus de 80 % en poids du résidu ALC des esters sont constitués d'ALC-c9t11, tandis que moins de 2% en poids du résidu ALC total sont constitués d'isomères ALC autres que c9t11 et t10c12 et les esters contiennent moins de 5 % en poids d'acides gras libres.

8. Procédé pour la préparation d'esters d'acide linoléique conjugué (ALC) et d'un alcool, comme définis dans la revendication 1, dans lequel l'acide linoléique conjugué libre est mis en réaction avec un alcool autorisé dans les produits alimentaires, comme défini dans la revendication 1, en présence de moins de 1,8 % en poids d'eau et d'une lipase.

9. Procédé pour la préparation d'esters d'ALC et d'un alcool, dans lequel les esters obtenus sont enrichis en ALC-C9t11 et dans lequel la réaction est réalisée au moyen de la conversion partielle d'un mélange d'ALC c9t11 et t10c12 dans un ratio de poids de 50:50 environ et d'un alcool autorisé dans les produits alimentaires du groupe défini dans la revendication 1 en présence d'une enzyme pouvant différencier les isomères ALC c9t11 et t10e12, les esters ALC enrichis en isomère ALC c9t11 étant ainsi isolés et les acides gras libres enrichis en isomère ALC t10c12 étant isolés en tant que réactifs non convertis.

10. Procédé selon les revendications 9 et 10, dans lequel le mélange de réaction brut obtenu est préparé de la façon suivante :
- addition d'abord d'une base diluée au mélange de réaction brut
- séparation de la phase aqueuse et de la phase organique
- lavage de la phase organique et ajustement du pH de cette phase au pH 5-7
- extraction de l'eau de cette phase par distillation

11. Procédé pour la préparation d'un mélange ALC libre enrichi en ALC-t10c12, dans lequel :
- la phase aqueuse du produit de réaction, obtenu conformément au procédé selon la revendication 10, est acidifiée à un pH < 3, de préférence < 1,5
- puis le mélange obtenu est séparé en une couche supérieure et en un résidu et
- la couche supérieure est lavée avec de l'eau et l'eau en est extraite.

12. Produits alimentaires comprenant un composant sain et gras, dans lesquels le composant sain est constitué d'un ou de plusicurs esters selon les revendications 1 à 6 ou de la composition d'esters selon la revendication 7.

13. Produits alimentaires selon la revendication 12, dans lesquels le produit alimentaire est choisi parmi le groupe des produits à tartiner (teneur en matière grasse de 10 à 90%) ; mayonnaise, fromage, crème glacée, enrobage de crème glacée ; enrobages ou garnitures de confiseries ; sauces et produits culinaires.

14. Produits alimentaires selon les revendications 12 et 13, dans lesquels le produit alimentaire contient dans le produit total de 0,5 à 20 % en poids des esters d'ALC selon les revendications 1 à 6 ou de la composition d'esters selon la revendication 7.

15. Procédé pour la préparation de triglycérides, dans lequel au moins un des résidus d'acides gras est un résidu d'acide linoléiquc conjugué, dans lequel un ester selon les revendications 1 à 6 ou la composition d'esters selon la revendication 7 est converti avec du glycérol ou avec une huile végétale en présence d'une base ou d'une enzyme, tandis que lors de la conversion du glycérol, le résidu d'alcool libéré issu des esters des esters d'ALC est extrait du mélange de réaction au cours de la conversion.
